# EUROPEAN PATENT SPECIFICATION

(11) **EP 1 888 011 B1**
(45) Date of publication and mention of the grant of the patent: **05.10.2011**
(21) Application number: 06770634.1
(22) Date of filing: 19.05.2006
(51) Int. Cl.: A61Q 1/02, A61Q 1/06

(54) **PIGMENT DISPERSION COMPOSITION COMPRISING AN ESTER BLEND AND MANUFACTURE THEREOF**
PIGMENTDISPERSIONSZUSAMMENSETZUNG MIT EINER ESTERMISCHUNG UND HERSTELLUNGSVERFAHREN DAFÜR
COMPOSITION DE DISPERSION DE PIGMENT RENFERMANT UN MELANGE D'ESTER ET PROCEDE DE PREPARATION DE LADITE COMPOSITION

(30) Priority: 03.06.2005 US 687235 P
(43) Date of publication of application: 20.02.2008
(73) Proprietor: LIPO CHEMICALS, INC., Paterson, NJ 07504 (US)
(72) Inventor: GREENBERG, Stephen, Mahwah, NJ 07430 (US); FRISCHLING, Louis, B., Lawrence, NY 11559 (US)
(74) Representative: Fiussello, Francesco
(86) International application number: PCT/US2006/019400
(87) International publication number: WO 2007/011449

(56) References cited:
- WO-A-91/16879
- US-A- 4 659 573
- US-A- 4 940 574
- US-A1- 2002 182 157

## Description

### CROSS-REFERENCE TO RELATED APPLICATION

This application claims the benefit of U.S. provisional Application Serial No. 60/687,235, filed June 3, 2005.

### FIELD OF THE INVENTION

The present invention is directed to a composition comprising a pigment dispersed in a liquid vehicle. More particularly, the present invention is directed to a dispersion of a pigment in an ester blend, wherein the amount of pigment dispersed in the ester blend is substantially greater than the amount of pigment dispersed in prior liquid vehicles. The present composition also exhibits improved flow properties which facilitates incorporation of the pigment dispersion into cosmetic formulations.

### BACKGROUND OF THE INVENTION

A number of cosmetic formulations, such as lipsticks, blushes, and skin coloring sticks, contain a relatively high amount of pigments in order to impart a desired color to the skin or lips. An ongoing problem in the design and manufacture of such cosmetic formulations is providing a homogeneous distribution of the pigment throughout the cosmetic formulation without costly and time-consuming process steps.

Typically, in the manufacture of lipsticks, pigments first are premixed with a vehicle, like castor oil, then this premix is added to a melt containing the remaining composition ingredients. The amount of pigment added to the vehicle is limited (a) by the capacity of the vehicle to wet and disperse the pigment to provide a stable dispersion and (b) by the flow properties of the pigment dispersion, e.g., the pigment dispersion requires a sufficient flow to allow introduction to and admixture with the remaining formulation ingredients. In many instances, the vehicle has a capacity to wet and disperse a relatively large amount of a pigment, but the flow properties then are poor and incorporation of the pigment dispersion into the cosmetic formulation is difficult and costly.

Therefore, a need exists in the art for a pigment dispersion containing a high percentage of a pigment, while retaining excellent flow properties for a facile incorporation into a cosmetic formulation.

### SUMMARY OF THE INVENTION

An aspect of the present invention is to maximize pigment dispersion in a liquid vehicle. Another aspect of the present invention is to provide a pigment dispersion containing a high percentage of pigment and that has excellent flow properties, which allows a facile introduction into a cosmetic formulation. The high amount of pigment in a present dispersion composition, and the excellent flow properties of the composition, reduce costs of manufacturing cosmetic formulations, and provide formulations that impart a high gloss after application.

In particular, a present pigment dispersion comprises an ester blend that permits incorporation of a higher level of a pigment into a pigment dispersion, i.e., a higher loading, than currently used vehicles, such as castor oil and tridecyl trimellitate. The ester blend of the present invention permits up to 1.5 times higher levels of pigment to be dispersed in the vehicle. In addition, a marked improvement in flow properties of the resulting pigment dispersion is observed.

### DETAILED DESCRIPTION OF THE PREFERRED EMBODIMENTS

Castor oil is the standard vehicle for pigment dispersions used in the personal care industry, especially lipsticks. Castor oil, however, has the above-discussed disadvantages with respect to the amount of pigment that can be dispersed and the flow properties of the resulting dispersion.

In accordance with the present invention, it has been found that a particular blend of esters is a surprisingly superior vehicle for dispersing pigments. The ester blend contains four esters, and the blend is a superior vehicle compared to any of the four individual esters, and to all combinations of two or three of the four esters. The present ester blend also is superior to castor oil and other currently used dispersants.

The ester blend utilized in the present invention is disclosed in U.S. Patent No. 4,659,573. The ester contains tridecyl trimellitate (TDTM) blended with dipentaerythritol hexacaprylate/hexacaprate (DPHC), tridecyl stearate (TDS), and neopentyl glycol dicaprylate/dicaprate (NPGC).

The amount of TDTM in the blend is about 5 to about 85 wt%, based on the ester blend, preferably about 5 to about 60 wt%, more preferably about 8 to about 50 wt%, most preferably about 10 to about 45 wt%, e.g., about 12 to about 40 wt%. The balance of the ester blend contains each of the three additional ester DPHC, TDS, and NPGC, each in an amount of about 2 to about 60 wt%, based on the ester blend. Preferable, DPHC is present in an amount of about 20% to about 55%, TDS is present in an amount of about 5% to about 20%, and NPGC is present in an amount of about 2% to about 15%, each by weight of the ester blend.

The following is an example of an ester blend useful in the present invention. The blend is prepared by simply admixing the individual esters until the blend is homogeneous.

| **Example 1** | |
|---|---|
| **Ester Component** | **Weight Percent** |
| TDTM | 39.70 |
| DPHC | 48.00 |
| TDS | 8.45 |
| NPGC | 3.85 |
| Total | 100.00 |

| | |
|---|---|
| The blend of Example 1 has a viscosity of about 340 S.S.U. | |

The above-described ester blend is used to disperse pigments, and provides (a) a higher pigment loading, i.e., up to 70% (by weight) pigment compared to 50-60% (by weight) for castor oil or TDTM alone, (b) a less viscous dispersion for facile incorporation of a high amount of pigment into a cosmetic formulation, and (c) a higher gloss and pigment development of the cosmetic formulation.

A composition of the present invention, comprises at least 50%, and more preferably at least 60%, by weight, of the pigment.

A composition of the present invention is prepared by simply admixing the pigment and the ester blend until homogeneous. The resulting composition has a sufficiently low viscosity to allow a fast and easy incorporation of the composition into a cosmetic formulation.

As used herein, the term "pigment" refers to any insoluble particles typically used in cosmetic formulations. The pigment can be an organic compound, an inorganic compound, or a mixture thereof, and can be deeply or vividly colored, such as a red used in a lipstick, or can be uncolored or mutely colored, such as titanium dioxide or earth colored particles for use in facial makeup compositions.

Pigments and dyes commonly used in the personal care area include, but are not limited to D&C Blue No. 4, D&C Brown No. 1, D&C Green No. 5, D&C Green No. 6, D&C Green No. 8, D&C Orange No. 4, D&C Orange No. 5, D&C Orange No. 10, D&C Orange No. 11, D&C Orange No. 17, D&C Red No. 6, D&C Red No. 7, D&C Red No. 8, D&C Red No. 9, D&C Red No. 17, D&C Red No. 19, D&C Red No. 21, D&C Red No. 22, D&C Red No. 27, D&C Red No. 28, D&C Red No. 30, D&C Red No. 31, D&C Red No. 33, D&C Red No. 34, D&C Red No.36, D&C Violet No. 2, D&C Yellow No. 7, D&C Yellow No. 8, D&C Yellow No. 10, D&C Yellow No. 11, Ext. D&C Violet No. 2, Ext. D&C Yellow No.7, FD&C Blue No.1, FD&C Green No. 3, FD&C Red No. 3, FD&C Red No. 4, FD&C Red No. 40, FD&C Yellow No. 5, FD&C Yellow No. 6;

Carmine, D&C Blue No. 1 Aluminum Lake, D&C Green No. 3 Aluminum Lake, D&C Orange No. 4 Aluminum Lake, D&C Orange No. 5 Aluminum Lake, D&C Orange No. 5 Zirconium Lake, D&C Orange No. 10 Aluminum Lake, D&C Orange No. 17 Lake, D&C Red No. 3 Aluminum Lake, D&C Red No. 4 Aluminum Lake, D&C Red No. 6 Aluminum Lake, D&C Red No. 6 Barium Lake, D&C Red No. 6 Barium/Strontium Lake, D&C Red No. 6 Potassium Lake, D&C Red No. 6 Strontium Lake, D&C Red No. 7 Aluminum Lake, D&C Red No. 7 Barium Lake, D&C Red No. 7 Calcium Lake, D&C Red No. 7 Calcium/Strontium Lake, D&C Red No. 7 Zirconium Lake, D&C Red No. 8 Sodium Lake, D&C Red No. 9 Aluminum Lake, D&C Red No. 9 Barium Lake, D&C Red No. 9 Barium/Strontium Lake, D&C Red No. 9 Zirconium Lake, D&C Red No. 19 Aluminum Lake, D&C Red No. 19 Barium Lake, D&C Red No. 19 Zirconium Lake, D&C Red No. 21 Aluminum Lake, D&C Red No. 21 Zirconium Lake, D&C Red No. 27 Aluminum Lake, D&C Red No. 27 Barium Lake, D&C Red No. 27 Calcium Lake, D&C Red No. 27 Zirconium Lake, D&C Red No. 30 Lake, D&C Red No. 31 Calcium Lake, D&C Red No. 33 Aluminum Lake, D&C Red No. 34 Calcium Lake, D&C Red No. 36 Lake, D&C Red No. 40 Aluminum Lake, D&C Yellow No. 5 Aluminum Lake, D&C Yellow No. 5 Zirconium Lake, D&C Yellow No. 6 Aluminum Lake, D&C Yellow No. 7 Zirconium Lake, D&C Yellow No. 10 Aluminum Lake, Ext. D&C Yellow No. 7 Aluminum Lake, FD&C Blue No. 1 Aluminum Lake, FD&C Red No. 3 Aluminum Lake, FD&C Red No. 4 Aluminum Lake, FD&C Yellow No. 5 Aluminum Lake, FD&C Yellow No. 6;

Aluminum Lake, Aluminum Powder, Annatto, Beta-Carotene, Bismuth Citrate, Bismuth Oxychloride, Bronze Powder, Caramel, Chlorophyllin-Copper Complex, Chromium Hydroxide Green, Chromium Hydroxide Greens, Copper Powder, Disodium EDTA-Copper, Ferric Ammonium Ferrocyanide, Guaiazulene, Guanine, Henna, Iron Oxides, Lead Acetate, Maganese Violet, Mica, Silver, Titanium Dioxide, Ultramarine Blue, Ultramarine Green, Ultramarine Pink, Ultramarine Red, Ultramarine Violet, and Zinc Oxide.

Preferred examples of dyes utilized.in the present cosmetic formulations, and that can be dispersed in the present ester blend, include but are not limited to inorganic pigments, such as yellow iron oxide, red iron oxide, black iron oxide, and titanium dioxide, and organic pigments, such as D&C Red #6 Lakes, D&C Red #7 Lakes, FD&C Yellow #5 Lakes, D&C Red #27 Lakes, D&C Red #21, and D&C Red #9.

### PIGMENT GRIND EVALUATIONS

### Esters and blends:

(a) Neopentyl Glycol Dicaprylate/Dicaprate (NPGC)
(b) Tridecyl Stearate (TDS)
(c) Tridecyl Trimellitate (TDTM)
(d) Dipentaerythrityl Hexacaprylate/Hexacaprate (DPC)
(e) Pentaerythrityl Tetracaprylate/Tetracaprate (PE-810)
(f) Castor Oil (CO)
(g) Blend containing four esters as follows (wt%):
   NPGC--3%
   TDS--9%
   TDTM--40%
   DPC--47%

### Pigments/Dyes--amount added to esters and ester blend (w/w) basis

Red 6 (Organic)--60%
Yellow 5 (Organic)--37.5%
Titanium Dioxide (Inorganic)--70%
Iron Oxide (Inorganic)--70%
All pigments were ground in a three-roll mill, and the resulting grinds were evaluated for wetting and flow characteristics.

Each ester present in blend (g) was evaluated alone (i.e., (a)-(d)) all combinations of two-and three-blended esters (a)-(d) also were evaluated. PE-810 and CO were evaluated alone only.

The pigment grinds prepared using blend (g) were wet better and as a result showed better flow properties than the other tested vehicles. Ester (f) and blend (g) were equivalent in wetting yellow 5. TDS, TDTM, DPC and PE-810 did not wet titanium oxide satisfactorily, resulting in powder rather than a grind. The superior flow properties demonstrated by blend (g) indicate better wetting and makes the grind easier to incorporate into formulations. Furthermore, better wetting typically results in a higher gloss of the grind.

## Claims

1. A composition comprising at least 50%, by weight, of pigment and an ester blend containing 5 to 85 wt% tridecyl trimellitate (TDTM), 2 to 60 wt% tridecyl stearate (TDS), 2 to 60 wt% dipentaerythritol hexacaprylate/hexacaprate (DPHC), and 2 to 60 wt% neopentyl glycol dicaprylate/dicaprate (NPGC).

2. The composition of claim 1 comprising at least 60%, by weight, of the pigment.

3. The composition of claim 1 wherein the ester blend comprises 10 to 45% TDTM, 20 wt% TDS, 2 to 15 wt% NPGC, 20 to 55 wt% DPHC, based on the blend.

4. The composition of claim 1 wherein the pigment is a deeply colored pigment.

5. The composition of claim 1 wherein the pigment is uncoloured or mutely colored.

6. The composition of claim 1 wherein the pigment is an organic compound, an inorganic compound, or a mixture thereof.

7. The composition of claim 6 wherein the pigment is selected from the group consisting of yellow iron oxide, red iron oxide, black iron oxide, titanium dioxide, D&C Red #6 Lakes, D&C Red #7 Lakes, FD&C Yellow #5 Lakes, D&C Red #27 Lakes, D&C Red #21, D&C Red #9, and mixtures thereof.

8. A method of forming a concentrated stable pigment dispersion comprising admixing 60 to 70 weight parts of a pigment with 30 to 40 weight parts of an ester blend containing 5 to 85% wt% TDTM, 2 to 60 wt% TDS, 2 to 60 wt% DPHC, and 2 to 60 wt% NPGC.

9. The method of claim 8 wherein the ester blend comprises 10 to 45% wt% TDTM, 5 to 20 wt% TDS, 2 to 15 wt% NPGC, 20 to 55 wt% DPHC, based on the blend.

10. A cosmetic formulation comprising a composition of claim 1.

11. The cosmetic formulation of claim 10 selected from the group consisting of a lipstick, a facial makeup, and a coloring stick.

## Patentansprüche

1. Zusammensetzung mit zumindest 50 Gew.-% Pigment und einer Esthermischung, die 5 bis 85 Gew.-% Tridecyltrimellitat (TDTM), 2 bis 60 Gew.-% Tridecylstearat (TDS), 2 bis 60 Gew.-% Dipentaerythritolhexacaprylat/hexacaprat (DPHC), und 2 bis 60 Gew.-% Neopentylglykoldicaprylat/dicaprat (NPGC) enthält.

2. Zusammensetzung nach Anspruch 1 mit zumindest 60 Gew.-% des Pigments.

3. Zusammensetzung nach Anspruch 1,
wobei die Estermischung 10 bis 45% TDTM, 20 Gew.-% TDS, 2 bis 15 Gew.-% NPCG, 20 bis 55 Gew.-% DPHC, basierend auf der Mischung, enthält.

4. Zusammensetzung nach Anspruch 1,
wobei das Pigment ein tief gefärbtes Pigment ist.

5. Zusammensetzung nach Anspruch 1,
wobei das Pigment ungefärbt oder gedämpft gefärbt ist.

6. Zusammensetzung nach Anspruch 1,
wobei das Pigment eine organische Verbindung, eine anorganische Verbindung oder eine Mischung davon ist.

7. Zusammensetzung nach Anspruch 6,
wobei das Pigment ausgewählt ist aus der Gruppe bestehend aus gelbem Eisenoxid, rotem Eisenoxid, schwarzem Eisenoxid, Titandioxid, D&C Rot #6 Lacke, D&C Rot #7 Lacke, FD&C Gelb #5 Lacke, D&C Rot #27 Lacke, D&C Rot #21, D&C Rot #9 und Mischungen davon.

8. Verfahren zum Bilden einer konzentrierten stabilen Pigmentdispersion mit Mischen von 60 bis 70 Gewichtsteilen eines Pigments mit 30 bis 40 Gewichtsteilen einer Estermischung, die 5 bis 85 Gew.-% TDTM, 2 bis 60 Gew.-% TDS, 2 bis 60 Gew.-% DPHC, und 2 bis 60 Gew.-% NPGC enthält.

9. Verfahren nach Anspruch 8,
wobei die Estermischung 10 bis 45 Gew.-% TDTM, 5 bis 20 Gew.-% TDS, 2 bis 15 Gew.-% NPGC, 20 bis 55 Gew.-% DPHC, basierend auf der Mischung, enthält.

10. Kosmetische Zubereitung mit einer Zusammensetzung nach Anspruch 1.

11. Kosmetische Zubereitung nach Anspruch 10 ausgewählt aus der Gruppe bestehend aus einem Lippenstift, einem Gesichts-Makeup und einem Färbestift.

## Revendications

1. Composition comprenant au moins 50 % en poids de pigment et un mélange d'esters contenant de 5 à 85 % en poids de trimellitate de tridécyle (TDTM), de 2 à 60 % en poids de stéarate de tridécyle (TDS), de 2 à 60 % en poids d'hexacaprylate/hexacaprate de dipentaérythritol (DPHC), et de 2 à 60 % en poids de dicaprylate/dicaprate de néopentylglycol (NPGC).

2. Composition selon la revendication 1, comprenant au moins 60% en poids du pigment.

3. Composition selon la revendication 1, dans laquelle le mélange d'esters comprend de 10 à 45% de TDTM, 20 % en poids de TDS, de 2 à 15 % en poids de NPGC, de 20 à 55% en poids de DPHC, sur la base du mélange.

4. Composition selon la revendication 1, dans laquelle le pigment est un pigment profondément coloré.

5. Composition selon la revendication 1, dans laquelle le pigment est non coloré ou a une coloration non marquée.

6. Composition selon la revendication 1, dans laquelle le pigment est un composé organique, un composé inorganique, ou un mélange de ceux-ci.

7. Composition selon la revendication 6, dans laquelle le pigment est choisi dans le groupe constitué par l'oxyde de fer jaune, l'oxyde de fer rouge, l'oxyde de fer noir, le dioxyde de titane, les laques D&C Red #6, les laques D&C Red #7, les laques FD&C Yellow #5, les laques D&C Red #27, D&C Red #21, D&C Red #9, et leurs mélanges.

8. Procédé pour former une dispersion de pigment stable concentrée, comprenant le mélange de 60 à 70 parties en poids d'un pigment avec 30 à 40 parties en poids d'un mélange d'esters contenant de 5 à 85 % en poids de TDTM, de 2 à 60 % en poids de TDS, de 2 à 60 % en poids de DPHC, et de 2 à 60 % en poids de NPGC.

9. Procédé selon la revendication 8, dans lequel le mélange d'esters comprend de 10 à 45 % en poids de TDTM, de 5 à 20 % en poids de TDS, de 2 à 15 % en poids de NPGC, de 20 à 55 % en poids de DPHC, sur la base du mélange.

10. Formulation cosmétique comprenant une composition de la revendication 1.

11. Formulation cosmétique selon la revendication 10, choisie dans le groupe constitué par un rouge à lèvres, un fond de teint pour le visage, et un bâton colorant.
